# EUROPÄISCHE PATENTSCHRIFT

(11) **EP 3 357 408 B1**
(45) Veröffentlichungstag und Bekanntmachung des Hinweises auf die Patenterteilung: **01.09.2021**
(21) Anmeldenummer: 18154849.6
(22) Anmeldetag: 02.02.2018
(51) Int. Cl.: A61B 1/267, A61B 1/005

(54) **LARYNGOSKOP UND ADAPTIVER SPATEL FÜR EIN LARYNGOSKOP**
LARYNGOSCOPE AND ADAPTIVE BLADE FOR A LARYNGOSCOPE
LARYNGOSCOPE ADAPTATIF ET SPATULE ADAPTATIVE POUR UN LARYNGOSCOPE

(30) Priorität: 02.02.2017 DE 102017102089
(43) Veröffentlichungstag der Anmeldung: 08.08.2018
(73) Patentinhaber: Karl Storz SE & Co. KG, 78532 Tuttlingen (DE)
(72) Erfinder: Efinger, Andreas, 78532 Tuttlingen (DE); Staud, Ralf, 78532 Tuttlingen (DE); Aleckner, Martina, 78532 Tuttlingen (DE)
(74) Vertreter: Wimmer, Stephan

(56) Entgegenhaltungen:
- EP-A1- 3 266 366
- EP-A1- 3 272 271
- WO-A2-2016/074894
- CN-A- 106 236 001
- DE-A1- 10 351 155

## Beschreibung

Die vorliegende Erfindung ist auf ein adaptives Laryngoskop, insbesondere ein adaptives Intubationslaryngoskop oder ein adaptives Laryngoskop für die Larynxchirurgie oder andere Aufgaben innerhalb der Hals-Nasen-Ohren-Heilkunde bzw. Oto-Rhino-Laryngologie (engl.: Otorhinolaryngology), und auf einen adaptiven Spatel (engl.: blade) für ein solches Laryngoskop bezogen.

Zur endotrachealen Intubation in Anästhesie, Notfallmedizin und Intensivmedizin sowie der Chirurgie des Kehlkopfs (Larynx) werden für eine Intubation oder für chirurgische Maßnahmen ein unverlegter Zugang zum Kehlkopf, den Stimmbändern und letztlich der Trachea benötigt. Dabei dient ein Laryngoskop dazu, die Zunge nach vorne bzw. rostral zu schieben. Ein Laryngoskop umfasst in der Regel einen mehr oder weniger gekrümmten Spatel, an dessen proximalem Ende in näherungsweise rechtem Winkel ein Griff angeordnet ist.

Um eine Anpassung an die Anatomie des Patienten zu ermöglichen, ist der Spatel in der Regel austauschbar. In einem Intubationsset befindet sich eine Vielzahl von Spateln unterschiedlicher Länge und unterschiedlicher Krümmung. Für verschiedene Anwendungen und/oder unterschiedlicher Vorlieben des medizinischen Personals stehen ferner unterschiedliche Bauformen zur Verfügung, beispielsweise nach Macintosh, Miller, Dörges und McCoy, letzterer mit einem bewegbaren distalen Ende.

Ein Laryngoskop mit einem verformbaren distalen Ende ist auch in WO 97/30626 beschrieben. Der Spatel 4 des Laryngoskops weist in einem mittleren Abschnitt 14 mehrere Schlitze 40 auf.

Die Schlitze 40 unterteilen den mittleren Abschnitt 14 in Segmente 42, die nur durch schmale, als Festkörpergelenke wirkende Stege miteinander verbunden sind.

In EP 1 040 999 A2 ist ein Bauteil zur Aufnahme von Kräften beschrieben, bei dem Streben 11, 11a gegenüberliegende Bereiche einer Außenhaut 12, 12a miteinander verbinden.

In EP 2 241 403 A1 ist ein Manipulatorwerkzeug mit zwei flexiblen Wangen 8, 10 beschrieben. Die Wangen 8, 10 sind am distalen Ende 6 des Manipulatorwerkzeugs 1 unmittelbar und im Übrigen durch mehrere Scharnierelemente 20 miteinander verbunden.

In DE 10 2007 026 721 A1 ist ein medizinisches Greifwerkzeug zum Halten von Körperbestandteilen beschrieben. Das medizinische Greifwerkzeug 1 umfasst mehrere Branchen 1 mit jeweils zwei gegenüberliegenden Wangen, zwischen denen verbindende Elemente verlaufen.

In CN 106236001 A ist ein flexibles Laryngoskop gezeigt, das zwei Holme 10, 20 aufzuweisen scheint. An einem Holm 20 ist ein Kamerakanal 50 angeordnet, der aus einem transparenten Material gebildet sein oder in den Holm 20 integriert sein kann (Absatz [0025], Figuren).

In DE 103 51 155 A1 ist ein Laryngoskop zur Intubation beschrieben. Das Laryngoskop umfasst einen Spatel 1, eine Lichtquelle 5, eine Kamera 6 und einen Bildschirm 8.

In DE 696 32 312 T2 ist eine Umhüllung für einen Kehlkopfspiegel beschrieben. Die Umhüllung kann nach einer Verwendung von dem Kehlkopfspiegel entfernt und entsorgt werden (Absatz [0010]).

In EP 3 266 366 A1 sind ein adaptives Laryngoskop und ein adaptiver Spatel für ein Laryngoskop beschrieben. Der adaptive Spatel (40) umfasst zwei biegbare Holme (50, 60), deren distale Enden miteinander mechanisch verbunden sind. Proximal des distalen Endes (44) des Spatels (40) sind die biegbaren Holme (50, 60) so miteinander mechanisch verbunden, dass sie relativ zueinander im Wesentlichen in ihrer Längsrichtung bewegbar sind. Dazu können die beiden biegbaren Holme (50, 60) mittels eines dünnen und biegbaren Verbindungsbauteils (70) miteinander verbunden sein.

Eine Aufgabe der vorliegenden Erfindung besteht darin, einen verbesserten adaptiven Spatel für ein Laryngoskop zu schaffen.

Diese Aufgabe wird durch die Gegenstände der unabhängigen Ansprüche gelöst.

Weiterbildungen sind in den abhängigen Ansprüchen angegeben.

Ein adaptiver Spatel für ein Laryngoskop umfasst ein proximales Ende, das mit einem Griffbauteil mechanisch verbindbar oder verbunden ist, um ein adaptives Laryngoskop zu bilden, einen ersten biegbaren Holm, der sich von dem proximalen Ende des adaptiven Spatels bis zu dessen distalem Ende erstreckt, einen zweiten biegbaren Holm, der sich von dem proximalen Ende des adaptiven Spatels bis zu dessen distalem Ende erstreckt, und eine Verbindungseinrichtung mit einem ersten Ende, das mit dem ersten biegbaren Holm gelenkig verbunden ist, und einem zweiten Ende, das mit dem zweiten biegbaren Holm gelenkig verbunden ist, wobei der erste biegbare Holm durch ein erstes Bauteil aus einem ersten Material und ein zweites Bauteil aus einem zweiten Material gebildet ist, wobei eine Grenzfläche zwischen dem ersten Bauteil und dem zweiten Bauteil sich über mindestens die Hälfte der Distanz zwischen dem proximalen Ende und dem distalen Ende des adaptiven Spatels erstreckt.

Der adaptive Spatel ist insbesondere Teil eines Laryngoskops oder zur Bildung eines Laryngoskops vorgesehen und ausgebildet, das zur Intubation oder für die Mikrolarynxchirurgie oder für andere Aufgaben innerhalb der Oto-Rhino-Laryngologie verwendbar ist. Das proximale Ende des adaptiven Spatels kann mit einem Griffbauteil dauerhaft - insbesondere für die gesamte vorgesehene Lebensdauer des Laryngoskops - und nicht zerstörungsfrei trennbar mechanisch verbunden sein. Insbesondere kann der adaptive Spatel teilweise oder ganz mit dem Griffbauteil einstückig ausgebildet sein, beispielsweise als gleichzeitig gefertigtes und monolithisches Gussteil aus Kunststoff, Metall oder einem anderen hinreichend elastischen Material. Alternativ kann am proximalen Ende des adaptiven Spatels eine Kupplungseinrichtung (beispielsweise in der Art einer Bajonett- bzw. Renkverbindung, einer Schraubverbindung, einer Rastverbindung) zur einmaligen oder wiederholbaren und zerstörungsfrei lösbaren Kopplung mit einem Griffbauteil vorgesehen sein.

Der erste biegbare Holm, der zweite biegbare Holm und die Verbindungseinrichtungen sind verschiedene Bereiche des adaptiven Spatels, die unterschiedliche Aufgaben und Funktionen übernehmen und unterschiedliche Eigenschaften aufweisen. Die Bereiche des adaptiven Spatels, die den ersten biegbaren Holm, den zweiten biegbaren Holm und die Verbindungseinrichtungen bilden, grenzen aneinander an und gehen in einander über. Von kleinen Übergangsbereichen zwischen dem ersten biegbaren Holm, dem zweiten biegbaren Holm und den Verbindungseinrichtungen, beispielsweise den erwähnten Festkörpergelenken, abgesehen sind der erste biegbare Holm, der zweite biegbare Holm und die Verbindungseinrichtungen klar von einander unterscheidbare Bereiche des Spatels.

Die beiden biegbaren Holme sind jeweils insbesondere bei den bei der vorgesehenen Verwendung auftretenden Kräften in Längsrichtung nicht oder nur geringfügig elastisch oder plastisch verformbar. Die elastische oder plastische Verformbarkeit der biegbaren Holme in Längsrichtung ist insbesondere so gering, dass eine Krümmung des adaptiven Spatels, die durch eine relative Verschiebung proximaler Enden der biegbaren Holme oder durch auf den adaptiven Spatel von außen einwirkende Kräfte bewirkt wird, im Vergleich zu idealisiert in Längsrichtung völlig unelastischen Holmen um nicht mehr als 50 % oder um nicht mehr als 20 % oder um nicht mehr als 10 % oder um nicht mehr als 5 % oder um nicht mehr als 2 % oder um nicht mehr als 1 % gemindert wird.

Die biegbaren Holme sind jeweils insbesondere nur in einer Richtung nennenswert elastisch und/oder plastisch biegbar und sind in einer dazu orthogonalen Richtung jeweils weitgehend biegesteif. Eine Biegbarkeit in einer ersten Richtung bzw. einer ersten Ebene und eine Biegesteifheit in einer dazu orthogonalen zweiten Richtung bzw. zweiten Ebene und eine geringe Verformbarkeit in Längsrichtung ist beispielsweise durch einen flachen Querschnitt eines Holms erzielbar, der in der ersten Richtung eine wesentliche kleinere Ausdehnung hat als in der zweiten Richtung. Beide biegbaren Holme sind jeweils insbesondere aus einem Polymer, einem anderen Kunststoff oder einem Metall gebildet.

Der erste biegbare Holm ist insbesondere vorgesehen und angeordnet, um bei der bestimmungsgemäßen Verwendung an der Zunge eines Patienten anzuliegen. Dazu ist der erste biegbare Holm insbesondere breit, flach und mit einer glatten zur Anlage an der Zunge vorgesehenen Oberfläche ausgebildet. Anstelle eines einzigen zweiten biegbaren Holms können zwei oder mehr, insbesondere parallel oder im Wesentlichen parallel zueinander angeordnete biegbare zweite Holme vorgesehen sein.

Ein Raumbereich zwischen zwei zweiten biegbaren Holmen kann zum Einführen eines Tubus, eines Endoskops und/oder eines anderen medizinischen Instruments vorgesehen sein. Dazu kann ein Führungskanal mit offenem oder geschlossenem Querschnitt vorgesehen sein. Dieser Führungskanal kann mit dem ersten biegbaren Holm oder mit dem zweiten biegbaren Holm oder mit einem oder mehreren von mehreren zweiten biegbaren Holmen starr verbunden, insbesondere einstückig ausgebildet sein.

Ferner kann eine Lichtquelle, beispielsweise eine Leuchtdiode, oder ein Lichtleitkabel zum Übertragen von Beleuchtungslicht, eine Kamera oder ein Bildsensor mit abbildender optischer Einrichtung oder ein flexibles Endoskop mit einem der biegbaren Holme - insbesondere an oder nahe dessen distalem Ende - verbindbar oder dauerhaft verbunden sein.

Eine Verbindung ist dauerhaft, wenn sie ausgelegt ist, um während eines mehrere medizinische Eingriffe umfassenden Zeitraums oder während der gesamten vorgesehenen Lebensdauer des Laryngoskops zu bestehen.

Am distalen Ende des adaptiven Spatels sind die distalen Enden der biegbaren Holme insbesondere mechanisch starr oder gelenkig miteinander verbunden. Im Fall einer gelenkigen Verbindung der biegbaren Holme am distalen Ende des adaptiven Spatels sind die distalen Enden der biegbaren Holme insbesondere so miteinander mechanisch verbunden, dass die distalen Enden der biegbaren Holme weder in Richtung parallel zu dem ersten biegbaren Holm noch in Richtung parallel zu dem zweiten biegbaren Holm relativ zueinander bewegbar sind.

Das erste Ende der Verbindungseinrichtung ist insbesondere durch ein erstes Festkörpergelenk mit dem ersten biegbaren Holm gelenkig verbunden. Das zweite Ende der Verbindungseinrichtung ist insbesondere durch ein zweites Festkörpergelenk mit dem zweiten biegbaren Holm verbunden. Zwischen ihren Enden ist die Verbindungseinrichtung insbesondere biegesteif ausgebildet oder zumindest deutlich weniger elastisch als die Festkörpergelenke an den Enden der Verbindungseinrichtung, so dass eine Verformung der Verbindungseinrichtung weitgehend oder vollständig auf die Festkörpergelenke begrenzt ist.

Der adaptive Spatel kann mehrere gleich oder ähnliche (sich insbesondere im Wesentlichen durch unterschiedliche Längen unterscheidende) Verbindungseinrichtungen aufweisen, wobei jede Verbindungseinrichtung den ersten biegebaren Holm und den zweiten biegbaren Holm miteinander verbindet. Wenn der erste biegbare Holm und der zweite biegbare Holm durch mehrere Verbindungseinrichtungen miteinander verbunden sind, nimmt die Länge der Verbindungseinrichtungen insbesondere von proximal nach distal ab. Wenn der adaptive Spatel mehrere zweite biegbare Holme aufweist, ist insbesondere jeder zweite biegbare Holm durch eine oder mehrere Verbindungseinrichtungen mit dem ersten biegbaren Holm verbunden.

Insbesondere werden das erste Bauteil durch einen ersten Körper aus einem ersten Material und das zweite Bauteil durch einen zweiten Körper aus einem zweiten Material gebildet.

Der erste biegbare Holm, der zweite biegbare Holm und die Verbindungseinrichtungen können zumindest teilweise aus den gleichen Materialien gebildet sein. Insbesondere ist sowohl der erste biegbare Holm als auch der zweite biegbare Holm jeweils aus zwei verschiedenen Materialien, nämlich aus dem ersten Material des ersten Bauteils (oder ersten Körpers) und dem zweiten Material des zweiten Bauteils (oder zweiten Körpers) gebildet. Beispielsweise kann ein erster Bereich des ersten Bauteils aus dem ersten Material einen ersten Teilbereich des ersten biegbaren Holms und ein zweiter Bereich des ersten Bauteils einen ersten Teilbereich des zweiten biegbaren Holms bilden, wobei ein erster Bereich des zweiten Bauteils aus dem zweiten Material einen zweiten Teilbereich des ersten biegbaren Holms, ein zweiter Bereich des zweiten Bauteils einen zweiten Teilbereich des zweiten biegbaren Holms und ein dritter Bereich des zweiten Bauteils die Verbindungseinrichtung oder dritte Bereiche des zweiten Bauteils die Verbindungseinrichtungen des adaptiven Spatels bilden. Sowohl das erste Bauteil als auch das zweite Bauteil kann also an mehreren Bereichen des adaptiven Spatels beteiligt sein. Dabei sind das erste Bauteil und das zweite Bauteil insbesondere eindeutig unterscheidbar, indem das erste Material des ersten Bauteils von dem zweiten Material des zweiten Bauteils verschieden ist. Entsprechend ist die Grenzfläche zwischen dem ersten Bauteil und dem zweiten Bauteil klar definiert.

Die Grenzfläche zwischen dem ersten Bauteil und dem zweiten Bauteil ist die Fläche, entlang derer das erste Bauteil und das zweite Bauteil unmittelbar aneinandergrenzen. Die Grenzfläche zwischen dem ersten Bauteil und dem zweiten Bauteil bildet gleichzeitig zumindest einen Teil der Oberfläche des ersten Bauteils und einen Teil der Oberfläche des zweiten Bauteils. Das erste Bauteil und das zweite Bauteil können an ihrer gesamten Grenzfläche miteinander stoffschlüssig verbunden sein. Alternativ oder zusätzlich können das erste Bauteil und das zweite Bauteil auf andere Weise gefügt sein.

Das erste Bauteil und das zweite Bauteil werden insbesondere bei verschiedenen und nacheinander stattfindenden Schritten eines Herstellungsverfahrens hergestellt. Beispielsweise wird zunächst das erste Bauteil hergestellt und dann das zweite Bauteil, beispielsweise durch Umspritzen des ersten Bauteils mit dem zweiten Bauteil oder Einbetten des ersten Bauteils in das zweite Material des zweiten Bauteils.

Ein adaptiver Spatel mit hier beschriebenen Merkmalen und Eigenschaften kann durch medizinisches Personal an die Anatomie eines Patienten angepasst werden. Alternativ oder zusätzlich kann ein adaptiver Spatel mit hier beschrieben Merkmalen und Eigenschaften bei der vorgesehenen Verwendung durch elastische und/oder plastische Verformung selbsttätig sich an die Anatomie eines Patienten teilweise oder vollständig anpassen, insbesondere an Krümmung, Form und/oder Größe der Zunge oder des Rachenraums des Patienten.

Diese Anpassung an die Anatomie des Patienten wird insbesondere ermöglicht durch die Biegeelastizität der beiden Holme und durch die Verbindungseinrichtung oder die Verbindungseinrichtungen. Die Verbindungseinrichtung bildet eine mechanische Verbindung der beiden biegeelastischen Holme derart, dass die beiden biegeelastischen Holme relativ zueinander im Wesentlichen in ihren Längsrichtungen bewegbar sind, ihre Abstände aber nicht wesentlich veränderbar sind. Dadurch wird ein lediglich lokales Zurückweichen des adaptiven Spatels ermöglicht. Beispielsweise kann der adaptive Spatel an einem konvexen Bereich der Oberfläche der Zunge eines Patienten zurückweichen, und trotzdem kann ein distaler Bereich des adaptiven Spatels an einem konkaven Bereich der Oberfläche der Zunge anliegen. Dabei kann der adaptive Spatel einen vergleichsweise gleichmäßigen Druck auf die Oberfläche der Zunge ausüben, und zwar sowohl in konkaven Bereichen als auch in konvexen Bereichen der Oberfläche der Zunge.

Deshalb kann ein solcher adaptiver Spatel für Patienten mit unterschiedlichen anatomischen Charakteristika verwendbar sein und damit die Anzahl der vorzuhaltenden Spatel deutlich verringern. Außerdem kann insbesondere in der Notfallmedizin Zeit gewonnen werden, indem nicht erst ein Spatel ausgewählt und mit dem Griffstück verbunden werden muss, sondern ein bereits mit einem Griffstück verbundener adaptiver Spatel bei seiner Verwendung an die Anatomie des Patienten angepasst wird und/oder sich anpasst.

Die Bildung des ersten biegbaren Holms durch zwei Bauteile aus zwei verschiedenen Materialien kann eine Verbesserung der mechanischen Eigenschaften des ersten biegbaren Holms ermöglichen. Insbesondere können erwünschte elastische Eigenschaften des ersten biegbaren Holms mit erwünschten atraumatischen Eigenschaften der Oberfläche des ersten biegbaren Holms vereint werden.

Bei einem adaptiven Spatel, wie er hier beschrieben ist, ist das erste Bauteil insbesondere ein Kernbauteil, wobei das zweite Bauteil ein Mantelbauteil, in das das erste Bauteil zumindest teilweise eingebettet ist, ist.

Bei einem adaptiven Spatel, wie er hier beschrieben ist, bedeckt das zweite Bauteil insbesondere mindestens ein Drittel oder mindestens die Hälfte oder mindestens zwei Drittel oder mindestens drei Viertel oder mindestens neun Zehntel der Oberfläche oder die gesamte Oberfläche des ersten Bauteils.

Das zweite Bauteil kann durch Umspritzen oder Umgießen des ersten Bauteils mit dem Material des zweiten Bauteils erzeugt sein. Eine erwünschte Wandstärke des zweiten Bauteils kann dabei insbesondere durch einen oder mehrere Abstandshalter (beispielsweise Ringe oder andere Einlegeteile) zwischen dem ersten Bauteil und der Wand der Gussform gewährleistet werden. Diese Abstandshalter weisen insbesondere das Material auf, aus dem das zweite Bauteil gebildet wird.

Indem das erste Bauteil in das zweite Bauteil zumindest teilweise eingebettet ist oder das zweite Bauteil zumindest einen Teil der Oberfläche des ersten Bauteils bedeckt, können das erste Bauteil für erwünschte mechanische Eigenschaften des ersten biegbaren Holms optimiert sein und gleichzeitig das zweite Bauteil atraumatische Eigenschaften des ersten biegbaren Holms und des adaptiven Spatels gewährleisten. Beispielsweise kann das erste Bauteil scharfe Kanten und Ecken aufweisen, die jedoch durch das zweite Bauteil bedeckt sind und deshalb nicht mit einem Patienten in Berührung kommen können.

Bei einem adaptiven Spatel, wie er hier beschrieben ist, weisen insbesondere das erste Material einen ersten Elastizitätsmodul E₁ und das zweite Material einen zweiten Elastizitätsmodul E₂ auf, wobei der erste Elastizitätsmodul E₁ größer als der zweite Elastizitätsmodul E₂ ist.

Insbesondere ist der erste Elastizitätsmodul E₁ mindestens um einen Faktor 10 größer als der zweite Elastizitätsmodul E₂. Dadurch können elastische Eigenschaften des ersten biegbaren Holms und des adaptiven Spatels weitgehend durch elastische Eigenschaften des ersten Bauteils definiert sein.

Bei einem adaptiven Spatel, wie er hier beschrieben ist, ist das erste Material insbesondere ein Metall.

Das erste Material ist insbesondere Federstahl 1.4310, Nitinol oder eine andere Formgedächtnis-Legierung oder ein anderes pseudoelastisches Material oder eine andere Legierung.

Bei einem adaptiven Spatel, wie er hier beschrieben ist, umfasst das erste Material insbesondere einen faserverstärkten Kunststoff.

Bei einem adaptiven Spatel, wie er hier beschrieben ist, umfasst das zweite Material insbesondere ein Silikon, einen Silikonkautschuk, ein Silikonelastomer, ein Silikonharz oder ein anderes Elastomer.

Mit Silikon ist insbesondere Poly(organo)siloxan gemeint. Ein Silikonharz ist insbesondere Polymetylsiloxan oder Polymetylphenylsiloxan.

Bei einem adaptiven Spatel, wie er hier beschrieben ist, ist das erste Bauteil insbesondere aus einem Blech oder einem anderen plattenförmigen Halbzeug gebildet.

Bei einem adaptiven Spatel, wie er hier beschrieben ist, weist die Dicke des Blechs insbesondere einen Wert im Bereich von 0,1 mm bis 0,4 mm oder im Bereich von 0,2 mm bis 0,3 mm auf.

Bei einem adaptiven Spatel, wie er hier beschrieben ist, weist das erste Bauteil insbesondere einen im Wesentlichen rechteckigen Querschnitt, dessen Breite mindestens den zwanzigfachen Wert oder mindestens den fünfzigfachen Wert oder mindestens den hundertfachen Wert seiner Höhe hat, auf.

Das angegebene Verhältnis zwischen Breite und Höhe des rechteckigen Querschnitts des ersten Bauteils gilt insbesondere an mehreren Stellen oder überall an dem ersten Bauteil.

Bei einem adaptiven Spatel, wie er hier beschrieben ist, weist das erste Bauteil insbesondere eine Ausnehmung auf.

Die Ausnehmung in dem ersten Bauteil ist insbesondere eine Öffnung bzw. ein Durchgangsloch, so dass das erste Bauteil im mathematischen Sinne mehrfach zusammenhängend ist. Alternativ reduziert die Ausnehmung lediglich lokal die Dicke bzw. den Querschnitt des ersten Bauteils. Das erste Bauteil kann mehrere Ausnehmungen aufweisen.

Eine oder mehrere Ausnehmungen an dem ersten Bauteil können seine Elastizität in einer vorbestimmten Richtung erhöhen. Dies kann - ähnlich wie ein großes Verhältnis zwischen Breite und Höhe eines Querschnitts des ersten Bauteils - eine hohe Biegeelastizität bei gleichzeitig niedriger Torsionselastizität des ersten Bauteils und damit des gesamten ersten biegbaren Holms ermöglichen.

Bei einem adaptiven Spatel, wie er hier beschrieben ist, erstreckt die Ausnehmung sich insbesondere orthogonal oder im Wesentlichen orthogonal zu der Längsrichtung des ersten biegbaren Holms.

Die Ausnehmung erstreckt sich in einer Richtung, wenn sie in dieser Richtung die größte lineare Abmessung aufweist. Eine Erstreckung der Ausnehmung orthogonal oder im Wesentlichen orthogonal zu der Längsrichtung des ersten biegbaren Holms kann eine große Biegeelastizität des ersten Bauteils und des ersten biegbaren Holms und gleichzeitig eine geringe Torsionselastizität - bezogen auf die Längsrichtung des ersten Holms - ermöglichen.

Bei einem adaptiven Spatel, wie er hier beschrieben ist, weist die Ausnehmung insbesondere in Längsrichtung des ersten biegbaren Holms eine erste maximale Abmessung und orthogonal zu der Längsrichtung des ersten biegbaren Holms eine zweite maximale Abmessung auf, wobei die erste maximale Abmessung nicht mehr als ein Drittel oder nicht mehr als ein Fünftel oder nicht mehr als ein Zehntel der zweiten maximalen Abmessung beträgt.

Die Ausnehmung ist insbesondere als Langloch ausgebildet.

Bei einem adaptiven Spatel, wie er hier beschrieben ist, ist das erste Bauteil insbesondere gewellt.

Insbesondere weist ein Längsschnitt des ersten Bauteils in einer Ebene parallel zu der Längsrichtung des ersten biegbaren Holms eine Wellenform mit einer oder mehreren Wellen oder Sicken auf. Die Welle oder die Wellen oder die Sicke oder die Sicken erstrecken sich insbesondere jeweils über die gesamte Breite oder im Wesentlichen über die gesamte Breite (insbesondere mindestens zwei Drittel oder mindestens vier Fünftel oder mindestens neun Zehntel der Breite) des ersten Bauteils. Eine oder mehrere Wellen oder Sicken des ersten Bauteils können erwünschte mechanische Eigenschaften des ersten Bauteils erzeugen, insbesondere eine hohe Biegeelastizität in einer Richtung und gleichzeitig eine geringe Torsionselastizität.

Bei einem adaptiven Spatel, wie er hier beschrieben ist, weisen insbesondere mehrere Ausnehmungen oder mehrere Wellen oder mehrere Sicken des ersten Bauteils unterschiedliche Abmessungen und/oder unterschiedliche Abstände auf.

Mehre Ausnehmungen oder mehrere Wellen oder mehrere Sicken des ersten Bauteils können unterschiedliche Breiten (insbesondere gemessen in Längsrichtung des ersten biegbaren Holms) und/oder unterschiedliche Längen (insbesondere gemessen in Richtung orthogonal zu der Längsrichtung des ersten biegbaren Holms) aufweisen. Mehrere Ausnehmungen können unterschiedliche Tiefen, mehrere Wellen oder Sicken können unterschiedliche Höhen aufweisen. Abmessungen und/oder Abstände der Ausnehmungen oder Wellen oder Sicken variieren insbesondere monoton oder streng monoton von proximal nach distal.

Beispielsweise nehmen die Abmessungen von Ausnehmungen oder Wellen oder Sicken von proximal nach distal zu, um eine von proximal nach distal zunehmende Elastizität des ersten Bauteils zu bewirken. Alternativ können die Abmessungen von Ausnehmungen oder Wellen oder Sicken von proximal nach distal abnehmen, um eine von proximal nach distal abnehmende Elastizität des ersten Bauteils zu bewirken.

Alternativ oder zusätzlich können die Abstände zwischen benachbarten Ausnehmungen oder Wellen oder Sicken von proximal nach distal zu- oder abnehmen, um eine von proximal nach distal variierende Elastizität des ersten Bauteils zu bewirken. Beispielsweise können die Abstände zwischen einer Vielzahl von Ausnehmungen, die als Langlöcher ausgebildet sind, von distal nach proximal abnehmen, wodurch auch die Breite von Stegen zwischen benachbarten Ausnehmungen von distal nach proximal abnimmt. Dadurch nimmt die Biegeelastizität des ersten Bauteils von distal nach proximal zu.

Bei einem adaptiven Spatel, wie er hier beschrieben ist, bildet das erste Bauteil insbesondere ferner zumindest einen Teil des zweiten biegbaren Holms.

Auch das zweite Bauteil kann einen Teil des zweiten biegbaren Holms bilden. Das zweite Bauteil kann das erste Bauteil auch im Bereich des zweiten biegbaren Holms teilweise oder weitgehend oder vollständig bedecken oder ummanteln oder umhüllen.

Bei einem adaptiven Spatel, wie er hier beschrieben ist, ist das erste Bauteil insbesondere durch Schneiden, Stanzen oder Ätzen aus einem Blech oder einem anderen plattenförmigen Halbzeug gebildet.

Das Blech oder das andere plattenförmige Halbzeug kann vor oder nach dem Stanzen, Schneiden oder Ätzen gebogen oder auf andere Weise plastisch verformt sein. Insbesondere können vor oder nach dem Schneiden, Stanzen oder Ätzen Wellen oder Sicken erzeugt worden sein.

Bei einem adaptiven Spatel, wie er hier beschrieben ist, weist das erste Bauteil an dem distalen Ende des adaptiven Spatels insbesondere einen gekrümmten Bereich auf und geht von dem ersten biegbaren Holm in den zweiten biegbaren Holm über.

Das erste Bauteil kann an dem distalen Ende des adaptiven Spatels eine oder zwei oder mehr gekrümmte Bereiche aufweisen und mit dieser oder diesen seine Grundrichtung insbesondere um insgesamt mindestens 150 Grad oder mindestens 160 Grad oder mindestens 170 Grad ändern. Die Gaußsche Krümmung des ersten Bauteils kann im gesamten Bereich des distalen Endes des adaptiven Spatels, einschließlich des gekrümmten Bereichs, null betragen oder von null abweichen.

Bei einem adaptiven Spatel, wie er hier beschrieben ist, umfasst ein Bereich des ersten Bauteils, der in dem zweiten biegbaren Holm angeordnet ist, insbesondere ein Rohr und einen teilweise in dem Rohr angeordneten Draht oder einen Draht mit von proximal nach distal variierendem Querschnitt oder weist auf andere Weise einen von proximal nach distal variierenden Querschnitt auf.

Bei einem adaptiven Spatel, wie er hier beschrieben ist, ist die Verbindungseinrichtung insbesondere durch das zweite Bauteil gebildet.

Die Verbindungseinrichtung oder mehrere Verbindungseinrichtungen oder alle Verbindungseinrichtungen des adaptiven Spatels können ausschließlich durch das zweite Bauteil gebildet sein. Alternativ kann die Verbindungseinrichtung oder können mehrere oder alle Verbindungseinrichtungen des adaptiven Spatels durch das erste Bauteil und durch das zweite Bauteil gebildet sein.

Alternativ können weitere Bauteile an der Verbindungseinrichtung oder den Verbindungseinrichtungen beteiligt sein.

Bei einem adaptiven Spatel, wie er hier beschrieben ist, umfasst der erste biegbare Holm insbesondere ferner ein drittes Bauteil aus dem ersten Material oder aus einem weiteren Material, wobei das zweite Bauteil zwischen dem ersten Bauteil und dem dritten Bauteil angeordnet ist.

Das dritte Bauteil kann aus einem weiteren Material gebildet sein, das von dem ersten Material und von dem zweiten Material verschieden ist. Das erste Bauteil, das zweite Bauteil und das dritte Bauteil können sandwichartig angeordnet sein. Dabei grenzen das erste Bauteil und das dritte Bauteil an gegenüberliegende Oberflächen des zweiten Bauteils an, und das erste Bauteil und das dritte Bauteil können zusammen einen Großteil (insbesondere mindestens die Hälfte, mindestens der Viertel oder mindestens neun zehntel) der Oberfläche des zweiten Bauteils bedecken.

Bei einem adaptiven Spatel, wie er hier beschrieben ist, weist das erste Bauteil insbesondere die Gestalt eines mehrfach durchbrochenen Rohrs auf.

Das erste Bauteil weist insbesondere die Gestalt eines mehrfach durchbrochenen zylindrischen oder konischen Rohrs mit beliebiger Grundfläche oder beliebigem Querschnitt auf. Grundfläche und/oder Querschnitte des Rohrs sind insbesondere kreisförmig, elliptisch oder rechteckig mit oder ohne abgerundeten (d.h. durch Viertelkreise ersetzten) Ecken.

Bei einem adaptiven Spatel, wie er hier beschrieben ist, weist das erste Bauteil insbesondere die Gestalt eines Ausschnitts einer Oberfläche eines verallgemeinerten Kegels auf.

Der verallgemeinerte Kegel weist eine beliebige, jedoch insbesondere konvexe Grundfläche auf. Die Grundfläche des verallgemeinerten Kegels ist beispielsweise kreisförmig, elliptisch, rechteckig, polygonal mit oder ohne abgerundeten Ecken.

Bei einem adaptiven Spatel, wie er hier beschrieben ist, weist das erste Bauteil insbesondere einen ersten Bereich, der in dem ersten biegbaren Holm angeordnet ist, einen zweiten Bereich, der in dem zweiten biegbaren Holm angeordnet ist, und mehrere bogenförmige Bereiche, die den ersten Bereich mit dem zweiten Bereich verbinden und in mehreren Verbindungseinrichtungen des adaptiven Spatels angeordnet sind, auf.

Der erste Bereich und der zweite Bereich sind jeweils insbesondere im Wesentlichen gerade oder in nur einer Richtung gekrümmte Stege. Die bogenförmigen Bereiche des ersten Bauteils, die den ersten Bereich mit dem zweiten Bereich verbinden, sind jeweils insbesondere kreisbogenförmig oder in einer ebenen Abwicklung des ersten Bauteils kreisbogenförmig.

Bei einem adaptiven Spatel, wie er hier beschrieben ist, sind insbesondere jeweils zwei kreisbogenförmige Bereiche des ersten Bauteils so angeordnet, dass sie einander an zwei Stellen schneiden.

Bei einem adaptiven Spatel, wie er hier beschrieben ist, ist das erste Bauteil insbesondere aus einem rohrförmig gebogenen und entlang einer sich in Längsrichtung des adaptiven Spatels erstreckenden Schweißnaht gefügten Blech gebildet.

Das Blech ist insbesondere mehrfach durchbrochen.

Ein adaptiver Spatel für ein Laryngoskop umfasst ein proximales Ende, das mit einem Griffbauteil mechanisch verbindbar oder verbunden ist, um ein adaptives Laryngoskop zu bilden, einen ersten biegbaren Holm, der sich von dem proximalen Ende des adaptiven Spatels bis zu dessen distalem Ende erstreckt, einen zweiten biegbaren Holm, der sich von dem proximalen Ende des adaptiven Spatels bis zu dessen distalem Ende erstreckt, und eine Verbindungseinrichtung mit einem ersten Ende, das mit dem ersten biegbaren Holm gelenkig verbunden ist, und einem zweiten Ende, das mit dem zweiten biegbaren Holm verbunden ist, wobei ein mehrfach durchbrochenes Rohr den ersten biegbaren Holm, den zweiten biegbaren Holm und die Verbindungseinrichtung bildet.

Der adaptive Spatel kann ähnliche Merkmale, Eigenschaften und Funktionen wie die oben beschriebenen adaptiven Spatel aufweisen. Insbesondere können an dem distalen Ende des adaptiven Spatels die biegbaren Holme miteinander mechanisch starr oder gelenkig verbunden sein. Die Enden von einer oder mehreren Verbindungseinrichtungen können jeweils durch Festkörpergelenke mit den biegbaren Holmen verbunden sein. Das mehrfach durchbrochene Rohr kann zylindrisch oder konisch mit beliebiger Grundfläche oder beliebigen Querschnitten sein. Die Grundfläche oder die Querschnitte des mehrfach durchbrochenen Rohrs sind insbesondere kreisförmig, elliptisch oder polygonal mit oder ohne abgerundeten Ecken.

Bei einem adaptiven Spatel, wie er hier beschrieben ist, weist das mehrfach durchbrochene Rohr insbesondere die Gestalt eines Ausschnitts einer Oberfläche eines verallgemeinerten Kegels auf.

Der verallgemeinerte Kegel weist eine beliebige, jedoch insbesondere konvexe Grundfläche auf. Die Grundfläche des verallgemeinerten Kegels ist beispielsweise kreisförmig, elliptisch, rechteckig, polygonal mit oder ohne abgerundeten Ecken.

Bei einem adaptiven Spatel, wie er hier beschrieben ist, weist das mehrfach durchbrochene Rohr insbesondere einen ersten Bereich, der in dem ersten biegbaren Holm angeordnet ist, einen zweiten Bereich, der in dem zweiten biegbaren Holm angeordnet ist, und mehrere bogenförmige Bereiche, die den ersten Bereich mit dem zweiten Bereich verbinden und mehreren Verbindungseinrichtungen des adaptiven Spatels bilden, auf.

Die bogenförmigen Bereiche des mehrfach durchbrochenen Rohrs, die den ersten Bereich mit dem zweiten Bereich verbinden, sind jeweils insbesondere kreisbogenförmig oder in einer ebenen Abwicklung des mehrfach durchbrochenen Rohrs kreisbogenförmig.

Bei einem adaptiven Spatel, wie er hier beschrieben ist, sind insbesondere jeweils zwei kreisbogenförmige Bereiche des mehrfach durchbrochenen Rohrs so angeordnet, dass sie einander an zwei Stellen schneiden.

Bei einem adaptiven Spatel, wie er hier beschrieben ist, ist mehrfach durchbrochene Rohr insbesondere aus einem rohrförmig gebogenen und entlang einer sich in Längsrichtung des adaptiven Spatels erstreckenden Schweißnaht gefügten Blech gebildet.

Ein adaptiver Spatel, wie er hier beschrieben ist, umfasst insbesondere ferner ein elastisches Überzugsbauteil zum Schutz des adaptiven Spatels vor Verschmutzung und anderen Umwelteinflüssen.

Das elastische Überzugsbauteil ist insbesondere nach jeder Verwendung des adaptiven Spatels austauschbar. Das elastische Überzugsbauteil kann eine elastische Folie oder ein elastisches Gewebe aufweisen.

Ein adaptiver Spatel, wie er hier beschrieben ist, weist insbesondere ferner einen Kanal, in den zumindest entweder ein Endoskop oder eine Lichtquelle oder ein anderes medizinisches Instrument eingeführt werden kann, auf.

Ein adaptiver Spatel, wie er hier beschrieben ist, kann für eine mehrmalige Verwendung und mehrmalige Sterilisation (insb. Dampfsterilisation im Autoklaven) oder für eine einmalige Verwendung und nachfolgende Entsorgung vorgesehen und ausgebildet sein.

Ein adaptives Laryngoskop umfasst einen adaptiven Spatel, wie er hier beschrieben ist, und ein Griffbauteil, das mit dem proximalen Ende des adaptiven Spatels mechanisch verbindbar oder verbunden ist.

Das adaptive Laryngoskop ist insbesondere ein Intubationslaryngoskop und/oder für eine Verwendung in der Mikrolarynxchirurgie oder für andere Anwendungen in der Oto-Rhino-Laryngologie vorgesehen.

Das Griffbauteil kann mit dem proximalen Ende des adaptiven Spatels derart mechanisch verbindbar oder verbunden sein, dass das Griffbauteil von dem proximalen Ende des adaptiven Spatels zerstörungsfrei lösbar oder nicht zerstörungsfrei lösbar ist.

### Kurzbeschreibung der Figuren

Nachfolgend werden Ausführungsformen anhand der beigefügten Figuren näher erläutert. Es zeigen:
- Figur 1: eine schematische axonometrische Darstellung eines adaptiven Laryngoskops;
- Figur 2: eine schematische axonometrische Darstellung eines ersten Bauteils eines adaptiven Spatels;
- Figur 3: eine schematische Darstellung eines weiteren ersten Bauteils eines adaptiven Spatels;
- Figur 4: eine schematische Darstellung eines weiteren ersten Bauteils eines adaptiven Spatels;
- Figur 5: eine schematische Darstellung eines adaptiven Spatels;
- Figur 6: eine schematische Darstellung eines weiteren adaptiven Spatels;
- Figur 7: eine schematische Darstellung eines weiteren adaptiven Spatels;
- Figur 8: eine schematische axonometrische Darstellung eines weiteren ersten Bauteils eines adaptiven Spatels;
- Figur 9: eine schematische Darstellung eines weiteren Intubationslaryngoskops;
- Figur 10: eine schematische Darstellung eines Bauteils des adaptiven Spatels des Intubationslaryngoskops aus Figur 9.

### Beschreibung der Ausführungsformen

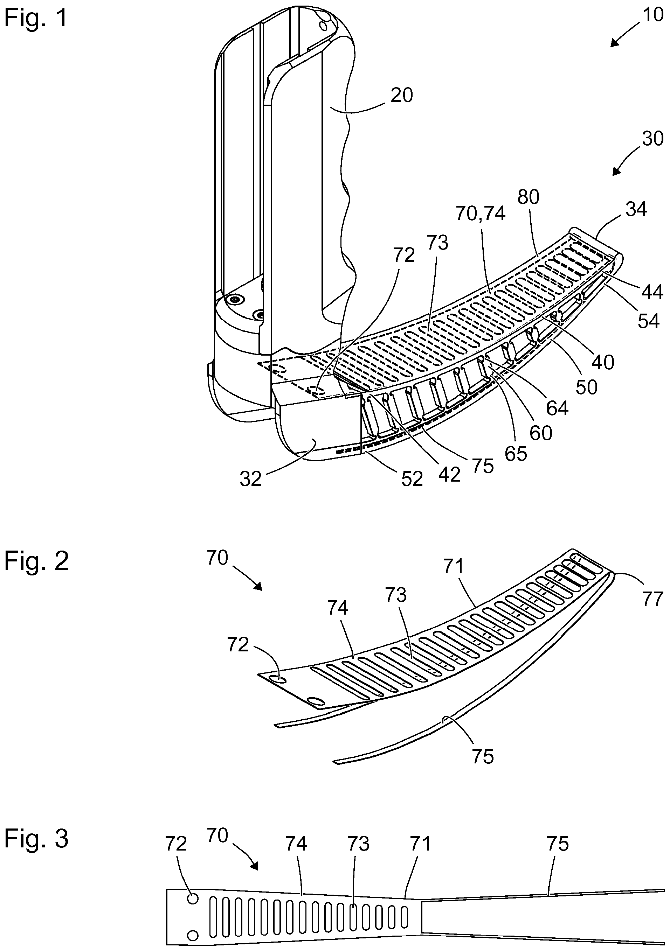

Figur 1 zeigt eine schematische axonometrische Darstellung eines adaptiven Intubationslaryngoskops 10 mit einem Griffbauteil 20 zum manuellen Halten und Führen des Intubationslaryngoskops 10. Ferner umfasst das adaptive Intubationslaryngoskop 10 einen adaptiven Spatel 30 mit einem proximalen Ende 32 und einem distalen Ende 34. Der adaptive Spatel 30 ist leicht gekrümmt und weist einen zum distalen Ende 34 hin sich vermindernden Querschnitt auf. Das proximale Ende 32 des adaptiven Spatels 30 ist derart mit dem Griffbauteil 20 mechanisch verbunden, dass der an das proximale Ende 32 angrenzende Bereich des adaptiven Spatels 30 mit dem Griffbauteil 20 einen Winkel von näherungsweise 90 Grad (im Bereich zwischen ca. 80 Grad und 130 Grad) einschließt.

In Figur 1 sind das Griffbauteil 20 und der adaptive Spatel 30 mit einander mechanisch verbunden dargestellt. Die Verbindung zwischen dem Griffbauteil 20 und dem adaptiven Spatel 30 erfolgt insbesondere mittels einer in Figur 1 nicht sichtbaren mechanischen Kupplung, die eine zerstörungsfreie Trennung des adaptiven Spatels 30 von dem Griffbauteil 20 ermöglicht. Alternativ können das Griffbauteil 20 und der adaptive Spatel 30 dauerhaft und nicht zerstörungsfrei trennbar miteinander verbunden sein.

Der adaptive Spatel 30 weist einen ersten biegbaren Holm 40 und zwei zweite biegbare Holme 50 auf. Bei der Darstellung in Figur 1 ist nur einer der beiden zweiten biegbaren Holme 50 vollständig sichtbar, der andere zweite biegbare Holm ist hinter dem ersten biegbaren Holm 40 verborgen.

Der erste biegbare Holm 40 weist im Wesentlichen einen breiten und flachen rechteckigen Querschnitt auf. Die in Figur 1 sichtbare und zur Anlage an einer Zunge eines Patienten vorgesehene Oberfläche des ersten biegbaren Holms 40 ist glatt oder weitgehend glatt.

Die zweiten biegbaren Holme 50 weisen jeweils einen im Wesentlichen quadratischen oder rechteckigen Querschnitt auf. Der Abstand der zweiten biegbaren Holme 50 entspricht im Wesentlichen der Breite des ersten biegbaren Holms 40.

Die zweiten biegbaren Holme 50 sind nahe dem proximalen Ende 32 des adaptiven Spatels 30 im Wesentlichen parallel zu den Rändern des ersten biegbaren Holms 40. Zum distalen Ende 34 des adaptiven Spatels 30 hin nehmen die Abstände der zweiten biegbaren Holme 50 von den Rändern des ersten biegbaren Holms 40 ab. Auch die Breite des ersten biegbaren Holms 40 und entsprechend der Abstand der beiden zweiten biegbaren Holme 50 voneinander nimmt zum distalen Ende 34 des adaptiven Spatels 30 hin etwas ab. Am distalen Ende 34 des adaptiven Spatels 30 sind das distale Ende 44 des ersten biegbaren Holms 40 und die distalen Enden der zweiten biegbaren Holme 50 miteinander mechanisch starr verbunden.

Die zweiten biegbaren Holme 50 sind mit den jeweils gegenüberliegenden Rändern des ersten biegbaren Holms 40 durch mehrere untereinander ähnliche Streben oder Verbindungseinrichtungen 60 verbunden. Das erste Ende 64 jeder Verbindungseinrichtung 60 ist mit dem ersten Holm 40 verbunden. Das zweite Ende 65 jeder Verbindungseinrichtung 60 ist mit dem zweiten Holm 50 verbunden. Je eine Verbindungseinrichtung 60, die den ersten biegbaren Holm 40 mit einem der zweiten biegbaren Holme 50 verbindet, ist parallel oder im Wesentlichen parallel zu einer weiteren Verbindungseinrichtung 60, die den ersten biegbaren Holm 40 mit dem anderen zweiten biegbaren Holm 50 verbindet.

Jede Verbindungseinrichtung 60 weist im Wesentlichen die Gestalt eines geraden Stabs mit zwischen den Enden 64, 65 im Wesentlichen konstantem Querschnitt auf. Jede Verbindungseinrichtung 60 weist in den Übergangsbereichen ihrer Enden 64, 65 zu dem ersten biegbaren Holm 40 bzw. zu dem zweiten biegbaren Holm 50 reduzierte Querschnitte und damit eine lokal erhöhte Biegeelastizität auf. Diese Übergangsbereiche mit reduzierten Querschnitten bilden Festkörpergelenke, die einer Veränderung der Winkel zwischen den Verbindungseinrichtungen 60 und den biegbaren Holmen 40, 50 nur geringe Rückstellkräfte entgegensetzen.

Die Verbindungseinrichtungen 60 und die Übergangsbereiche zwischen den Enden 64, 65 der Verbindungseinrichtungen und den biegbaren Holmen 40, 50 sind so dimensioniert, dass bei den bei der vorgesehenen Verwendung des adaptiven Intubationslaryngoskops 10 auftretenden Kräften keine nennenswerte Änderung der Längen der Verbindungseinrichtungen stattfindet. Dadurch sind die Abstände der zweiten biegbaren Holme 50 von den jeweils gegenüberliegenden Rändern des ersten biegbaren Holms 40 im Wesentlichen unveränderbar festgelegt bzw. definiert. Die zweiten biegbaren Holme 50 können jedoch relativ zu den gegenüberliegenden Rändern des ersten biegbaren Holms 40 verschoben werden. Dabei ist die Relativbewegung der zweiten biegbaren Holme 50 und des ersten biegbaren Holms 40 orthogonal oder im Wesentlichen orthogonal zu den Verbindungseinrichtungen 60 und damit bei der in Figur 1 angedeuteten Konfiguration parallel oder im Wesentlichen parallel zu den biegbaren Holmen 40, 50.

Der adaptive Spatel 30 des adaptiven Intubationslaryngoskops 10 ist im Wesentlichen aus einem ersten Bauteil 70 als Kernbauteil und einem zweiten Bauteil 80 als Mantelbauteil gebildet. Das erste Bauteil 70 ist bei dem dargestellten Beispiel vollständig von dem zweiten Bauteil 80 umgeben. Das erste Bauteil 70 ist deshalb - soweit das zweite Bauteil 80 nicht aus einem transparenten Material besteht - nicht sichtbar und in Figur 1 nur mit gestrichelten Linien angedeutet.

Das erste Bauteil 70 ist sowohl in dem ersten biegbaren Holm 40 als auch in den zweiten biegbaren Holmen 50 angeordnet, bildet also - zusammen mit dem zweiten Bauteil 80 - den ersten biegbaren Holm 40 und die zweiten biegbaren Holme 50. Das erste Bauteil 70 ist bei dem dargestellten Beispiel nicht in den Verbindungseinrichtungen 60 angeordnet, die Verbindungseinrichtungen 60 werden also ausschließlich durch das zweite Bauteil 80 gebildet.

Das erste Bauteil 70 ist bei dem dargestellten Beispiel aus einem geschnittenen, gestanzten oder gefrästen und davor oder danach gebogenen Blech gebildet.

An dem proximalen Ende 32 des adaptiven Spatels 30 weist das erste Bauteil 70 zwei Befestigungslöcher 72 auf. Die Befestigungslöcher 72 ermöglichen eine Befestigung des ersten Bauteils 70 an dem Griffbauteil 20 des Intubationslaryngoskops 10 oder an einer Kupplungs- oder Verbindungseinrichtung am proximalen Ende 32 des adaptiven Spatels 30, wobei die Kupplungs- oder Verbindungseinrichtung ihrerseits mit dem Griffbauteil 20 dauerhaft oder lösbar verbunden werden kann.

Der in dem ersten biegbaren Holm angeordnete Bereich 74 des ersten Bauteils 70 ist im wesentlichen stegförmig und weist mehrere Ausnehmungen 73 auf. Die Ausnehmungen 73 sind insbesondere Durchgangsbohrungen. Bei dem dargestellten Beispiel ist jede einzelne Ausnehmung 73 schmal und länglich mit zwei parallelen geraden Seiten und zwei abgerundeten Enden. Die Ausnehmungen 73 sind parallel zueinander und orthogonal zu der Längsrichtung des ersten biegbaren Holms 40 angeordnet.

Die Ausnehmungen 73 beeinflussen die Elastizität des ersten Bauteils 70 und des ersten Holms 40. Insbesondere erhöhen die Ausnehmungen 73 die Biegeelastizität des in dem ersten biegbaren Holm 40 angeordneten Bereichs 74 des ersten Bauteils 70 und damit des ersten biegbaren Holms 40. Die Ausnehmungen 73 erhöhen aber die Elastizität des ersten Bauteils 70 und des erste biegbaren Holms 40 vor allem hinsichtlich einer Verformung in der durch das Griffbauteil 20 und den adaptiven Spatel 30 aufgespannten Ebene, vereinfachen also eine Vergrößerung oder Verkleinerung der Krümmung des adaptiven Spatels 30. Die Elastizität des ersten Bauteils 70 und damit des ersten biegbaren Holms 40 und somit des gesamten adaptiven Spatels 30 hinsichtlich einer Torsion wird durch die Ausnehmungen 73 hingegen in einem vergleichsweise geringeren Maße erhöht. Die Ausnehmungen 73 verbessern somit durch ihre längliche Gestalt und ihre Orientierung orthogonal zu der Längsrichtung des ersten Holms 40 das Verhältnis zwischen Biegeelastizität und Torsionselastizität des adaptiven Spatels 30.

Figur 2 zeigt eine schematische axonometrische Darstellung des ersten Bauteils 70 des anhand der Figur 1 dargestellten adaptiven Spatels 30. Die Blickrichtung, aus der das erste Bauteil 70 in Figur 2 dargestellt ist, entspricht derjenigen der Figur 1. Da das erste Bauteil 70 in Figur 2 ohne die Ummantelung durch das zweite Bauteil 80 dargestellt ist, ist das erste Bauteil 70 vollständig sichtbar und deshalb in durchgezogenen Linien dargestellt.

In Figur 2 ist erkennbar, dass die Abstände zwischen benachbarten den Ausnehmungen 73 in dem für den ersten biegbaren Holm 40 vorgesehenen Bereich 74 des ersten Bauteils 70 von proximal (in den Figuren 1 und 2: links) nach distal (in den Figuren 1 und 2: rechts) abnehmen. Dies kann zu einer von proximal nach distal zunehmenden Elastizität des adaptiven Spatels beitragen.

Bei dem dargestellten Beispiel nehmen die Abstände der Enden der Ausnehmungen 73 von den Längsrändern 71 des ersten Bauteils 70 zu dem proximalen Ende hin ab. Auch dies kann die Elastizität des adaptiven Spatels beeinflussen, insbesondere seine Elastizität nahe dem proximalen Ende erhöhen.

In Figur 2 sind ferner beide Bereiche 75 des ersten Bauteils 70, die zur Anordnung in je einem zweiten biegbaren Holm 50 des adaptiven Spatels 30 (vgl. Figur 1) vorgesehen sind, sichtbar. Diese Bereiche 75 des ersten Bauteils 70 weisen jeweils die Gestalt eines im Wesentlichen nur in einer Richtung gekrümmten Stegs auf.

Das erste Bauteil 70 weist in dem Bereich, der zur Anordnung an dem distalen Ende 34 des adaptiven Spatels 30 (vgl. Figur 1) vorgesehen ist, einen gekrümmten Bereich 77 mit einem durch die Abmessungen des distalen Endes 34 des adaptiven Spatels 30 bedingten kleinen Krümmungsradius auf.

Figur 3 zeigt eine schematische Darstellung eines weiteren ersten Bauteils 70, das in einigen Merkmalen, Eigenschaften und Funktionen dem anhand der Figuren 1 und 2 dargestellten ersten Bauteil 70 ähnelt. Das in Figur 3 gezeigte erste Bauteil 70 ist beispielsweise für einen adaptiven Spatel und für ein adaptives Intubationslaryngoskops, die den anhand der Figur 1 dargestellten ähneln oder im Übrigen gleichen, vorgesehen.

Das erste Bauteil 70 ist in Figur 3 in einem ungekrümmten Zustand, wie er beispielsweise unmittelbar nach dem Schneiden oder Stanzen aus einem Blech vorliegt, gezeigt.

Bei dem in Figur 3 gezeigten ersten Bauteil 70 nehmen die in Richtung orthogonal zu der Längsrichtung des ersten Bauteils 70 (und damit des adaptiven Spatels, für den das erste Bauteil 70 vorgesehen ist) gemessenen Längen der Ausnehmungen 73 von proximal nach distal im gleichen Maße ab, wie die Breite des zur Anordnung in dem ersten biegbaren Holm des adaptiven Spatels vorgesehenen Bereichs 74. Die Abstände der Enden der Ausnehmungen 73 von den Längsrändern 71 des ersten Bauteils 70 sind deshalb im Wesentlichen konstant.

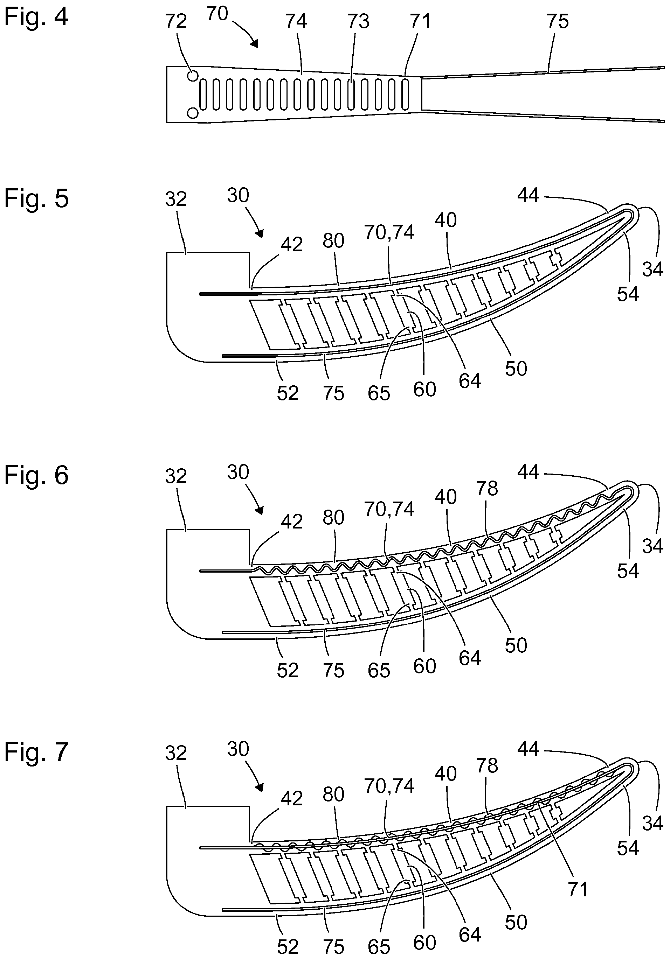

Figur 4 zeigt eine schematische Darstellung eines weiteren ersten Bauteils 70, das in einigen Merkmalen, Eigenschaften und Funktionen den anhand der Figuren 1 bis 3 dargestellten ersten Bauteilen für oder von adaptive Spatel ähnelt. Die Art der Darstellung in Figur 4 entspricht derjenigen der Figur 3. Nachfolgend sind insbesondere Merkmale, Eigenschaften und Funktionen des ersten Bauteils 70 beschrieben, in denen dieses sich von den anhand der Figuren 1 bis 3 dargestellten ersten Bauteilen unterscheidet.

Das in Figur 4 gezeigte erste Bauteil 70 unterscheidet sich von den anhand der Figuren 1 bis 3 dargestellten ersten Bauteilen insbesondere dadurch, dass die in Richtung orthogonal zu der Längsrichtung des ersten Bauteils 70 gemessenen Längen der Ausnehmungen 73 gleich sind. Die Abstände der Enden der Ausnehmungen 73 von den Längsrändern 71 des ersten Bauteils 70 nehmen deshalb von proximal nach distal ab. Entsprechend steigt die Elastizität des ersten Bauteils 70 von proximal nach distal an.

Figur 5 zeigt eine schematische Darstellung eines weiteren adaptiven Spatels 30, der in einigen Merkmalen, Eigenschaften und Funktionen dem anhand der Figur 1 dargestellten adaptiven Spatel ähnelt. Die Zeichenebene der Figur 5 ist parallel zu der Längsrichtung des adaptiven Spatels 30 und wäre parallel zu einem Griffbauteil, wenn dieses in der vorgesehenen Weise mit dem adaptiven Spatel 30 mechanisch verbunden wäre.

Figur 6 zeigt eine schematische Darstellung eines weiteren adaptiven Spatels, der in einigen Merkmalen, Eigenschaften und Funktionen den anhand der Figuren 1 und 5 dargestellten adaptiven Spateln ähnelt. Die Art der Darstellung in Figur 6 entspricht derjenigen der Figur 5. Nachfolgend sind insbesondere Merkmale, Eigenschaften und Funktionen beschrieben, in denen der in Figur 6 gezeigte adaptive Spatel 30 sich von den anhand der Figuren 1 bis 5 dargestellten adaptiven Spateln unterscheidet.

Der in Figur 6 gezeigte adaptive Spatel unterscheidet sich von den anhand der Figuren 1 bis 5 dargestellten adaptiven Spateln insbesondere dadurch, dass das erste Bauteil 70 keine Ausnehmungen, sondern stattdessen Wellen oder Sicken 78 aufweist. Jede einzelne Welle oder Sicke 78 erstreckt sich in Richtung orthogonal zu der Längsrichtung des ersten biegbaren Holms 40 und damit orthogonal zu der Zeichenebene der Figur 6. Die Wellen oder Sicken 78 erhöhen die Biegeelastizität des in dem ersten biegbaren Holm 40 angeordneten Bereichs 74 des ersten Bauteils 70 (im Vergleich zu einem ersten Bauteil 70, das weder Wellen oder Sicken 78 noch Ausnehmungen aufweist) und damit die Biegeelastizität des ersten biegbaren Holms 40 und des gesamten adaptiven Spatels 30. Gleichzeitig erhöhen die Wellen oder Sicken 78 die Torsionssteifigkeit des in dem ersten biegbaren Holm 40 angeordneten Bereichs 74 des ersten Bauteils 70 und damit auch die Torsionssteifigkeit des ersten Holms 40 und des gesamten adaptiven Spatels 30.

Figur 7 zeigt eine schematische Darstellung eines weiteren adaptiven Spatels, der in einigen Merkmalen, Eigenschaften und Funktionen den anhand der Figuren 1, 5 und 6 dargestellten adaptiven Spateln ähnelt. Die Art der Darstellung in Figur 7 entspricht denjenigen der Figuren 5 und 6. Nachfolgend sind insbesondere Merkmale, Eigenschaften und Funktionen des in Figur 7 gezeigten adaptiven Spatels 30 beschrieben, in denen dieser sich von den anhand der Figuren 1, 5 und 6 dargestellten adaptiven Spateln unterscheidet.

Der in Figur 7 gezeigte adaptive Spatel 30 unterscheidet sich insbesondere von dem anhand der Figur 6 dargestellten adaptiven Spatel vor allem dadurch, dass die Wellen oder Sicken 78 nicht bis zu den Längsrändern 71 des ersten Bauteils 70 reichen. Die Längsränder 71 des ersten Bauteils 70 sind deshalb ebenso glatt und weisen eine ebenso kontinuierliche Krümmung auf, wie bei den anhand der Figuren 1 bis 5 dargestellten adaptiven Spateln.

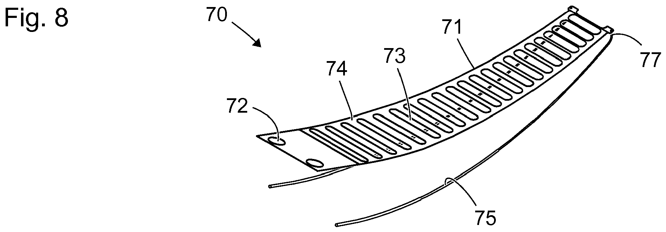

Figur 8 zeigt eine schematische axonometrische Darstellung einer alternativen Ausgestaltung des ersten Bauteils 70 des anhand der Figur 1 dargestellten adaptiven Spatels 30. Die Blickrichtung, aus der das erste Bauteil 70 in Figur 8 gezeigt ist, entspricht derjenigen der Figuren 1 und 2, im Übrigen entspricht die Art der Darstellung derjenigen der Figur 2. Insbesondere ist das erste Bauteil 70 in Figur 8 ohne die Ummantelung durch das zweite Bauteil 80 und deshalb in durchgezogenen Linien dargestellt.

Das in Figur 8 gezeigte erste Bauteil 70 ähnelt in einigen Eigenschaften, Merkmalen und Funktionen dem anhand der Figur 2 dargestellten ersten Bauteil. Nachfolgend sind insbesondere Merkmale, Eigenschaften und Funktionen beschrieben, in denen das in Figur 8 gezeigte erste Bauteil 70 sich von dem anhand der Figur 2 dargestellten unterscheidet.

Das in Figur 8 gezeigte erste Bauteil 70 unterscheidet sich von dem anhand der Figur 2 dargestellten ersten Bauteil insbesondere dadurch, dass es nicht aus einem einzigen Stück Blech gebildet ist. Vielmehr umfasst das erste Bauteil 70 einen zur Anordnung in dem ersten biegbaren Holm 40 (vgl. Figur 2) vorgesehenen Bereich 74 und zwei an diesen gefügte Bereiche 75, die zur Anordnung in den zweiten biegbaren Holmen 50 vorgesehen sind. Der zur Anordnung in dem ersten biegbaren Holm vorgesehene Bereich 74 ähnelt dem des anhand der Figur 2 dargestellten ersten Bauteils. Jeder zur Anordnung in einem zweiten biegbaren Holm vorgesehener Bereich 75 weist eine von proximal (in Figur 8: links unten) nach distal (in Figur 8: rechts oben) abnehmende Querschnittsfläche auf. Die distalen Enden der zur Anordnung in den zweiten biegbaren Holmen vorgesehenen Bereiche 75 sind in Bohrungen am distalen Ende des zur Anordnung im ersten Holm vorgesehenen Bereichs 74 eingesetzt und beispielsweise durch Laserschweißen mit dem Bereich 74 verbunden.

Bei dem in Figur 8 gezeigten ersten Bauteil 70 weist jeder für die Anordnung in einem zweiten biegbaren Holm vorgesehener Bereiche 75 einen von proximal nach distal stufenförmig abnehmenden Querschnitt auf. Die Biegeelastizität jedes für die Anordnung in einem zweiten biegbaren Holm vorgesehenen Bereichs 75 nimmt deshalb von proximal nach distal zu.

Insbesondere ist jeder für die Anordnung in einem zweiten biegbaren Holm vorgesehener Bereiche 75 aus einem ersten Rohr, einem zweiten Rohr, das dünner und länger ist als das erste Rohr, und einem Draht gefertigt. Das zweite Rohr ist in dem ersten Rohr angeordnet und ragt aus diesem nach distal heraus. Der Draht ist in dem zweiten Rohr angeordnet und ragt aus diesem nach distal heraus. Die proximalen Enden des zweiten Rohrs und des Drahts sind insbesondere an dem proximalen Ende des ersten Rohrs angeordnet. Die Querschnitte sind so gewählt, dass der Draht in dem zweiten Rohr und das zweite Rohr in dem ersten Rohr jeweils spielarm geführt sind. Das erste Rohr, das zweite Rohr und der Draht können durch Kleben, Schweißen oder Löten oder auf andere Weise miteinander verbunden sein.

Alternativ kann jeder für die Anordnung in einem zweiten biegbaren Holm vorgesehener Bereich 75 aus einem Stück Draht gefertigt sein, dessen Querschnitt von proximal nach distal abnimmt. Beispielsweise ist der Querschnitt des Drahts an seinem proximalen Ende kreisförmig und an seinem distalen Ende kreissegmentförmig. Ein derartiger Querschnittsverlauf kann ausgehend von einem Draht mit durchgehend kreisförmigem Querschnitt beispielsweise durch Fräsen entlang einer Ebene oder einer anderen Fläche, die nicht parallel zu dem Draht ist, erzeugt werden. Der Ausgangsdurchmesser des Drahts beträgt beispielsweise 0,8 mm.

Die Fläche und/oder die Höhe (gemessen in Richtung der Krümmung des Bereichs 75 in der in Figur 8 dargestellten Konfiguration des ersten Bauteils 70) des Querschnitts jedes Bereichs 75 kann von proximal nach distal kontinuierlich oder diskontinuierlich variieren, um eine für die vorgesehene Verwendung optimale Biegeelastizität des Bereichs 75 zu erzeugen. Die Fläche und/oder die Höhe kann monoton oder streng monoton, beispielsweise einer affin linearen Gleichung entsprechend variieren.

Bei dem anhand der Figur 8 dargestellten ersten Bauteil 70 weist der für die Anordnung in dem ersten biegbaren Holm vorgesehene Bereich 74 Ausnehmungen 73 auf, die in Form, Größe und Anordnung denen des anhand der Figur 2 dargestellten ersten Bauteils entsprechen. Alternativ und abweichend von der Darstellung in Figur 8 kann der für die Anordnung in dem ersten biegbaren Holm vorgesehene Bereich 74 des ersten Bauteils 70 Ausnehmungen anderer Größe und Anordnung (wie beispielsweise anhand der Figuren 3 und 4 dargestellt) und/oder Wellen oder Sicken (wie beispielsweise anhand der Figuren 6 und 7 dargestellt) aufweisen.

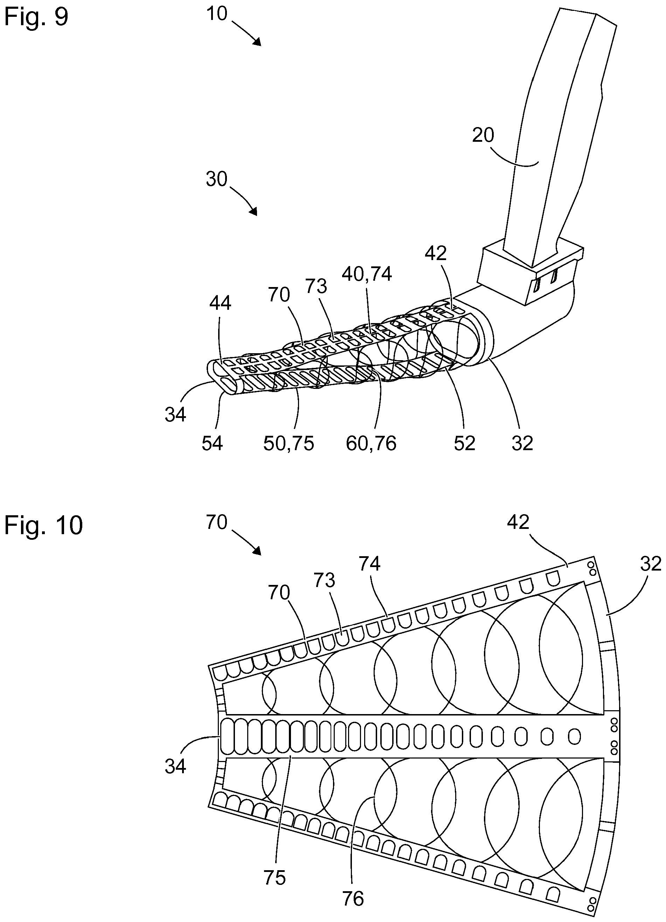

Figur 9 zeigt eine schematische axonometrische Darstellung eines weiteren adaptiven Intubationslaryngoskops 10, das in einigen Merkmalen, Eigenschaften und Funktionen dem anhand der Figur 1 dargestellten adaptiven Intubationslaryngoskop ähnelt, und dessen adaptiver Spatel 30 in einigen Merkmalen, Eigenschaften und Funktionen den anhand der Figuren 1 bis 8 dargestellten adaptiven Spateln ähnelt. Nachfolgend sind insbesondere Merkmale, Eigenschaften und Funktionen beschrieben, in denen der adaptive Spatel 30 des in Figur 9 gezeigten adaptiven Intubationslaryngoskops 10 sich von den anhand der Figuren 1 bis 8 dargestellten adaptiven Spateln unterscheidet.

Der adaptive Spatel 30 des in Figur 9 gezeigten Intubationslaryngoskops unterscheidet sich von den anhand der Figuren 1 bis 8 dargestellten adaptiven Spateln insbesondere dadurch, dass sowohl ein erster biegbarer Holm 40 und ein zweiter biegbarer Holm 50 als auch Verbindungseinrichtungen 60 zwischen den biegbaren Holmen 40, 50 aus einem einzigen rohrförmigen Bauteil 70 gebildet sind. Dieses rohrförmige Bauteil 70 weist die Gestalt eines konischen Rohrs mit zahlreichen Durchbrechungen bzw. Ausnehmungen auf. Zwischen den Durchbrechungen verbleibende stegförmige Flächen oder Bereiche 74, 75, 76 bilden den ersten biegbaren Holm 40, den zweiten biegbaren Holm 50 und die Verbindungseinrichtungen 60. Der erste biegbare Holm 40 ist insbesondere zur Anlage an der Oberfläche der Zunge eines Patienten vorgesehen.

Im Unterschied zu den anhand der Figuren 1 bis 8 dargestellten adaptiven Spateln ist an einer nicht zur Anlage an der Zunge eines Patienten vorgesehenen Seite des adaptiven Spatels 30 nur ein zweiter biegbarer Holm 50 vorgesehen, der bei dem dargestellten Beispiel jedoch genauso breit ist wie der erste biegbare Holm 40. Ähnlich wie bei dem anhand der Figur 1 dargestellten adaptiven Spatel und den anhand der Figuren 2 bis 4 und 8 dargestellten ersten Bauteilen sind in dem ersten biegbaren Holm 40 mehrere Ausnehmungen 73 vorgesehen. Entsprechend sind auch in dem zweiten biegbaren Holm 50 mehrere Ausnehmungen vorgesehen. Die Ausnehmungen 73 in den biegbaren Holmen 40, 50 bzw. den Bereichen 74, 75 des rohrförmigen Bauteils 70, die die biegbaren Holme 40, 50 bilden, beeinflussen die Biegeelastizität und die Torsionssteifigkeit des rohrförmigen Bauteils 70 und damit des adaptiven Spatels 30.

Die Torsionssteifigkeit wird ferner durch die bogenförmigen Bereiche 76 des rohrförmigen Bauteils 70, das den adaptiven Spatel 30 bildet, und die als Verbindungseinrichtungen 60 wirken, beeinflusst. Die bogenförmigen Bereiche 76 schneiden einander paarweise jeweils in zwei Schnittpunkten.

Das proximale Ende 32 und das distale Ende 34 des adaptiven Spatels 30 sind jeweils durch ringförmig geschlossene Bereiche des rohrförmigen Bauteils 70 gebildet. An dem proximalen Ende 32 und an dem distalen Ende 34 des adaptiven Spatels 30 sind die länglichen Querschnitte des rohrförmigen Bauteils 70 erkennbar. Der Querschnitt des rohrförmigen Bauteils 70 nimmt von dem proximalen Ende 32 bis zu dem distalen Ende 34 monoton ab.

Bei dem dargestellten Beispiel weisen die Querschnitte des rohrförmigen Bauteils 70 jeweils zwei einander gegenüberliegende halbkreisbogenförmige und dazwischen zwei einander gegenüberliegende gerade Abschnitte auf.

Abweichend von der Darstellung in Figur 9 kann das rohrförmige Bauteil 70 als erstes Bauteil des adaptiven Spatels 30 teilweise oder vollständig von einem zweiten Bauteil ummantelt sein. In diesem Fall weist das zweite Bauteil insbesondere aufgrund der elastischen Eigenschaften seines Materials und/oder seiner Wandstärken und sonstigen Abmessungen eine höhere Elastizität auf als das erste Bauteil 70, so dass die elastischen Eigenschaften des adaptiven Spatels 30 überwiegend oder weitgehend durch die elastischen Eigenschaften des rohrförmigen Bauteils 70 bestimmt sind. Das zweite Bauteil kann den Kontakt von Oberflächen des ersten Bauteils 70 mit einem Patienten verhindern. Scharfe Kanten oder mangelnde Biokompatibilität des Materials des rohrförmigen Bauteils 70 bedingen deshalb keine Einschränkungen bei der Verwendung des adaptiven Spatels 30.

Figur 10 zeigt eine schematische Darstellung einer Abwicklung des rohrförmigen Bauteils 70 des anhand der Figur 9 dargestellten adaptiven Spatels 30 und damit eine Darstellung eines bereits geschnittenen oder gestanzten, jedoch noch nicht gebogenen Blechs, aus dem das rohrförmige Bauteil 70 des anhand der Figur 9 dargestellten adaptiven Spatels 30 zu bilden ist.

In Figur 10 sind die in der Abwicklung kreisbogenförmigen Bereiche 76, die die Verbindungseinrichtungen 60 des adaptiven Spatels 30 (vgl. Figur 9) bilden, gut erkennbar. Jeweils zwei in entgegengesetzter Richtung gekrümmte kreisbogenförmige Bereiche 76 schneiden einander in jeweils zwei Schnittpunkten näherungsweise orthogonal.

Bei allen anhand der Figuren 1 bis 10 dargestellten adaptiven Spateln und ihren Varianten sind das erste Bauteil bzw. das rohrförmige Bauteil 70 insbesondere aus einem Blech aus Federstahl, Nitinol oder einem anderen Metall gebildet und das zweite Bauteil insbesondere aus einem Elastomer gebildet. Alternativ kann das erste Bauteil 70 aus einem faserverstärkten Kunststoff oder aus einem anderen Kunststoff gebildet sein. Insbesondere bei einer Ausgestaltung ähnlich der anhand der Figuren 9 und 10 dargestellten, bei der das erste Bauteil auch die Verbindungseinrichtungen 60 bildet, kann das zweite Bauteil entfallen. Der adaptive Spatel kann als Zweikomponenten-Spritzgussteil gebildet sein.

Von proximal nach distal variierende elastische Eigenschaften der anhand der Figuren dargestellten adaptiven Spatel und ihrer Varianten können durch variierende Abmessungen mehrerer Ausnehmungen oder variierende Abmessungen von Wellen oder Sicken eingestellt werden. Alternativ oder zusätzlich können von proximal nach distal variierende elastische Eigenschaften durch variierende Wandstärken erzeugt werden. Dies gilt insbesondere dann, wenn das erste Bauteil nicht aus einem Blech, sondern durch ein Gussverfahren oder durch ein additives Verfahren hergestellt ist.

Bei allen anhand der Figuren dargestellten Intubationslaryngoskopen kann die Verbindung zwischen Griffbauteil 20 und adaptivem Spatel 30 durch eine Rastverbindung herstellbar sein. Bei allen anhand der Figuren dargestellten Intubationslaryngoskopen kann abweichend von den Darstellungen in den Figuren ein Kanal zum Einführen eines Endoskops, einer Lichtquelle und/oder eines anderen Instruments vorgesehen sein. Ferner können abweichend von den Darstellungen in den Figuren eine Kamera, eine Bildübertragungseinrichtung und/oder eine Lichtquelle in den adaptiven Spatel integriert sein.

### Bezugszeichen

- 10: Intubationslaryngoskop
- 20: Griffbauteil des Intubationslaryngoskops 10
- 30: adaptiver Spatel des Intubationslaryngoskops 10 oder für das Intubationslaryngoskop 10
- 32: proximales Ende des adaptiven Spatels 30
- 34: distales Ende des adaptiven Spatels 30
- 40: erster biegbarer Holm des adaptiven Spatels 30
- 42: proximales Ende des ersten biegbaren Holms 40
- 44: distales Ende des ersten biegbaren Holms 40
- 50: zweiter biegbarer Holm des adaptiven Spatels 30
- 52: proximales Ende des zweiten biegbaren Holms 50
- 54: distales Ende des zweiten biegbaren Holms 50
- 60: Verbindungseinrichtung oder Strebe
- 64: erstes Ende der Verbindungseinrichtung 60, mit dem ersten biegbaren Holm 40 verbunden
- 65: zweites Ende der Verbindungseinrichtung 60, mit dem zweiten biegbaren Holm 50 verbunden
- 70: erstes Bauteil oder rohrförmiges Bauteil oder Kernbauteil
- 71: Längsrand des ersten Bauteils 70
- 72: Befestigungsloch in dem ersten Bauteil 70
- 73: Ausnehmung in dem für den ersten biegbaren Holm 40 angeordneten Bereich 74 des ersten Bauteils 70
- 74: in dem ersten biegbaren Holm 40 angeordneter oder den ersten biegbaren Holm 40 bildender Bereich des ersten Bauteils 70
- 75: in dem zweiten biegbaren Holm 50 angeordneter oder den zweiten biegbaren Holm 50 bildender Bereich des ersten Bauteils 70
- 76: bogenförmiger Bereich des ersten Bauteils 70 zwischen dem ersten biegbaren Holm 40 und dem zweiten biegbaren Holm 50
- 77: gekrümmter Bereich oder Knick des ersten Bauteils 70 an dem distalen Ende 34 des adaptiven Spatels 30
- 78: Welle oder Sicke in dem ersten Bauteil 70
- 80: zweites Bauteil

## Patentansprüche

1. **Adaptiver Spatel** (30) für ein Laryngoskop (10), mit:
einem **proximalen Ende** (32), das mit einem Griffbauteil (20) mechanisch verbindbar oder verbunden ist, um ein adaptives Laryngoskop (10) zu bilden;
einem **ersten biegbaren Holm** (40), der sich von dem proximalen Ende (32) des adaptiven Spatels (30) bis zu dessen distalem Ende (34) erstreckt;
einem **zweiten biegbaren Holm** (50), der sich von dem proximalen Ende (32) des adaptiven Spatels (30) bis zu dessen distalem Ende (34) erstreckt;
einer **Verbindungseinrichtung** (60) mit einem ersten Ende (64), das mit dem ersten biegbaren Holm (40) gelenkig verbunden ist, und einem zweiten Ende (65), das mit dem zweiten biegbaren Holm (50) gelenkig verbunden ist,
wobei der erste biegbare Holm (40) durch ein **erstes Bauteil** (70) aus einem ersten Material und ein **zweites Bauteil** (80) aus einem zweiten Material gebildet ist,
wobei eine **Grenzfläche** zwischen dem ersten Bauteil (70) und dem zweiten Bauteil (80) sich über mindestens die Hälfte der Distanz zwischen dem proximalen Ende (32) und dem distalen Ende (34) des adaptiven Spatels (30) erstreckt,
wobei das erste Bauteil (70) ein **Kernbauteil** ist,
wobei das zweite Bauteil (80) ein **Mantelbauteil,** in das das erste Bauteil (70) zumindest teilweise eingebettet ist, ist.

2. Adaptiver Spatel (30) nach dem vorangehenden Anspruch, bei dem
das erste Material einen ersten Elastizitätsmodul E₁ und das zweite Material einen zweiten Elastizitätsmodul E₂ aufweisen, wobei der **erste Elastizitätsmodul E₁ größer** als der **zweite Elastizitätsmodul E₂** ist.

3. Adaptiver Spatel (30) nach einem der vorangehenden Ansprüche, bei dem
das erste Bauteil (70) aus einem **Blech** oder einem anderen plattenförmigen Halbzeug gebildet ist.

4. Adaptiver Spatel (30) nach einem der vorangehenden Ansprüche, bei dem
das erste Bauteil (70) eine **Ausnehmung** (73) aufweist.

5. Adaptiver Spatel (30) nach dem vorangehenden Anspruch, bei dem
die Ausnehmung (73) sich **orthogonal** oder im Wesentlichen orthogonal zu der Längsrichtung des ersten biegbaren Holms (40) erstreckt.

6. Adaptiver Spatel (30) nach einem der vorangehenden Ansprüche, bei dem
das erste Bauteil (70) **gewellt** ist.

7. Adaptiver Spatel (30) nach einem der Ansprüche 4 bis 6, bei dem mehrere Ausnehmungen (73) oder Wellen (78) oder mehrere Sicken des ersten Bauteils (70) zumindest entweder unterschiedliche Abmessungen oder unterschiedliche Abstände aufweisen.

8. Adaptiver Spatel (30) nach einem der vorangehenden Ansprüche, bei dem
das **erste Bauteil** (70) ferner zumindest einen Teil des **zweiten biegbaren Holms** (50) bildet.

9. Adaptiver Spatel (30) nach dem vorangehenden Anspruch, bei dem ein Bereich (75) des ersten Bauteils (70), der in dem zweiten biegbaren Holm (50) angeordnet ist, ein **Rohr** und einen teilweise in dem Rohr angeordneten **Draht** oder einen **Draht mit** von proximal nach distal **variierendem Querschnitt** umfasst oder auf andere Weise einen von proximal nach distal variierenden Querschnitt aufweist.

10. Adaptiver Spatel (30) nach einem der vorangehende Ansprüche, bei dem:
der erste biegbare Holm (40) ferner ein **drittes Bauteil** aus dem ersten Material oder aus einem weiteren Material umfasst,
das zweite Bauteil (80) zwischen dem ersten Bauteil (70) und dem dritten Bauteil angeordnet ist.

11. Adaptiver Spatel (30) nach einem der vorangehenden Ansprüche, bei dem
das erste Bauteil (70) die Gestalt eines mehrfach **durchbrochenen Rohrs** aufweist.

12. Adaptiver Spatel (30) nach dem vorangehenden Anspruch, bei dem
das erste Bauteil (70) die Gestalt eines Ausschnitts einer Oberfläche eines verallgemeinerten Kegels aufweist.

13. Adaptiver Spatel (30) nach einem der Ansprüche 11 und 12, bei dem das erste Bauteil (70)
einen **ersten Bereich** (74), der in dem ersten biegbaren Holm (40) angeordnet ist,
einen **zweiten Bereich** (75), der in dem zweiten biegbaren Holm (50) angeordnet ist, und
mehrere bogenförmige Bereiche (76), die den ersten Bereich (74) mit dem zweiten Bereich (75) verbinden und in mehreren Verbindungseinrichtungen (60) des adaptiven Spatels (30) angeordnet sind, aufweist.

14. **Adaptives Laryngoskop** (10) mit:
einem adaptiven **Spatel** (30) nach einem der vorangehenden Ansprüche;
einem **Griffbauteil** (20), das mit dem proximalen Ende (32) des adaptiver Spatels (30) mechanisch verbindbar oder verbunden ist.

## Claims

1. Adaptive blade (30) for a laryngoscope (10), comprising:
a proximal end (32), which is mechanically connectable or connected to a handle (20)in order to form an adaptive laryngoscope (10);
a first flexible bar (40), which extends from the proximal end (32) of the adaptive blade (30) to the distal end (34) thereof;
a second flexible bar (50), which extends from the proximal end (32) of the adaptive blade (30) to the distal end (34) thereof;
a connecting structure (60) with a first end (64), which is connected to the first flexible bar (40) in an articulated manner, and with a second end (65), which is connected to the second flexible bar (50) in an articulated manner,
wherein the first flexible bar (40) is formed by a first member (70) made of a first material and a second member (80) made of a second material,
wherein an interface between the first member (70) and the second member (80) extends over at least half the distance between the proximal end (32) and the distal end (34) of the adaptive blade (30),
wherein the first member (70) is a core member,
wherein the second member (80) is a jacket member in which the first member (70) is at least partially embedded.

2. Adaptive blade (30) according to the preceding claim, wherein
the first material has a first elastic modulus E₁ and the second material has a second elastic modulus E₂, wherein the first elastic modulus E₁ is greater than the second elastic modulus E₂.

3. Adaptive blade (30) according to one of the preceding claims, wherein
the first member is formed from a metal sheet or another plate-shaped semi-finished product.

4. Adaptive blade (30) according to one of the preceding claims, wherein
the first member (70) has a recess (73).

5. Adaptive blade (30) according to the preceding claim, wherein
the recess (73) extends orthogonally or substantially orthogonally with respect to the longitudinal direction of the first flexible bar (40).

6. Adaptive blade (30) according to one of the preceding claims, wherein
the first member (70) is undulated.

7. Adaptive blade (30) according to one of the claims 4 through 6, wherein several recesses (73) or waves (78) or several beads of the first member (70) have at least either different dimensions or different spacings.

8. Adaptive blade (30) according to one of the preceding claims, wherein
the first member (70) moreover forms at least a part of the second flexible bar (50).

9. Adaptive blade (30) according to the preceding claim, wherein a region (75) of the first member (70), which region is arranged in the second flexible bar (50), comprises a tube and a wire partially arranged in the tube, or a wire with a cross section varying from proximal to distal, or in some other way has a cross section varying from proximal to distal.

10. Adaptive blade (30) according to one of the preceding claims, wherein:
the first flexible bar (40) moreover comprises a third member made from the first material or from a further material,
the second member (80) is arranged between the first member (70) and the third member.

11. Adaptive blade (30) according to one of the preceding claims, wherein
the first member (70) has the shape of a multiply perforated tube.

12. Adaptive blade (30) according to the preceding claim, wherein
the first member (70) has the shape of a cut-out of a surface of a generalized cone.

13. Adaptive blade (30) according to one of the claims 11 and 12, wherein the first member (70) has
a first region (74), which is arranged in the first flexible bar (40),
a second region (75), which is arranged in the second flexible bar (50) , and
several arc-shaped regions (76), which connect the first region (74) to the second region (75) and are arranged in several connecting structures (60) of the adaptive blade (30).

14. Adaptive laryngoscope (10) comprising:
an adaptive blade (30) according to one of the preceding claims;
a handle (20), which is mechanically connectable or connected to the proximal end (32) of the adaptive blade (30).

## Revendications

1. Spatule adaptative (30) pour un laryngoscope (10), comportant :
une extrémité proximale (32), qui est reliée ou peut être reliée mécaniquement à un composant de préhension (20), afin de former un laryngoscope adaptatif (10) ;
une première barre flexible (40), qui s'étend de l'extrémité proximale (32) de la spatule adaptative (30) à son extrémité distale (34) ;
une deuxième barre flexible (50), qui s'étend de l'extrémité proximale (32) de la spatule adaptative (30) à son extrémité distale (34) ;
un dispositif de liaison (60) doté d'une première extrémité (64), qui est reliée de manière articulée à la première barre flexible (40), et d'une deuxième extrémité (65), qui est reliée de manière articulée à la deuxième barre flexible (50),
la première barre flexible (40) étant formée par un premier composant (70) constitué d'un premier matériau et un deuxième composant (80) constitué d'un deuxième matériau,
une surface limite entre le premier composant (70) et le deuxième composant (80) s'étendant sur au moins la moitié de la distance entre l'extrémité proximale (32) et l'extrémité distale (34) de la spatule adaptative (30),
le premier composant (70) étant un composant de noyau,
le deuxième composant (80) étant un composant d'enveloppe dans lequel le premier composant (70) est encastré au moins partiellement.

2. Spatule adaptative (30) selon la revendication précédente, dans laquelle
le premier matériau présente un premier module d'élasticité E1 et le deuxième matériau présente un deuxième module d'élasticité E2, le premier module d'élasticité E1 étant supérieur au deuxième module d'élasticité E2.

3. Spatule adaptative (30) selon l'une des revendications précédentes, dans laquelle
le premier composant (70) est formé à partir d'une tôle ou d'un autre produit semi-fini en forme de plaque.

4. Spatule adaptative (30) selon l'une des revendications précédentes, dans laquelle
le premier composant (70) comprend un évidement (73).

5. Spatule adaptative (30) selon la revendication précédente, dans laquelle
l'évidement (73) s'étend de manière orthogonale ou sensiblement orthogonale à la direction longitudinale de la première barre flexible (40).

6. Spatule adaptative (30) selon l'une des revendications précédentes, dans laquelle
le premier composant (70) est ondulé.

7. Spatule adaptative (30) selon l'une des revendications 4 à 6, dans laquelle plusieurs évidements (73) ou ondulations (78) ou plusieurs nervures du premier composant (70) présentent au moins soit différentes dimensions soit différents espacements.

8. Spatule adaptative (30) selon l'une des revendications précédentes, dans laquelle
le premier composant (70) forme en outre au moins une partie de la deuxième barre flexible (50).

9. Spatule adaptative (30) selon la revendication précédente, dans laquelle une région (75) du premier composant (70), qui est disposée dans la deuxième barre flexible (50), comporte un tube et un fil disposé partiellement dans le tube ou un fil de section transversale variant de l'extrémité proximale vers l'extrémité distale, ou présente d'une autre manière une section transversale variant de l'extrémité proximale vers l'extrémité distale.

10. Spatule adaptative (30) selon l'une des revendications précédentes, dans laquelle :
la première barre flexible (40) comporte en outre un troisième composant constitué du premier matériau ou d'un autre matériau,
le deuxième composant (80) est disposé entre le premier composant (70) et le troisième composant.

11. Spatule adaptative (30) selon l'une des revendications précédentes, dans laquelle
le premier composant (70) présente la forme d'un tube percé plusieurs fois.

12. Spatule adaptative (30) selon la revendication précédente, dans laquelle
le premier composant (70) présente la forme d'une section d'une surface d'un cône généralisé.

13. Spatule adaptative (30) selon l'une des revendications 11 et 12, dans laquelle le premier composant (70) comprend
une première région (74), qui est disposée dans la première barre flexible (40),
une deuxième région (75), qui est disposée dans la deuxième barre flexible (50), et
plusieurs régions arquées (76), qui relient la première région (74) à la deuxième région (75) et sont disposées dans plusieurs dispositifs de liaison (60) de la spatule adaptative (30).

14. Laryngoscope adaptatif (10) comportant :
une spatule adaptative (30) selon l'une des revendications précédentes ;
un composant de préhension (20), qui est relié ou peut être relié mécaniquement à l'extrémité proximale (32) de la spatule adaptative (30).
